# EUROPÄISCHE PATENTANMELDUNG

(11) **EP 1 607 058 A1**
(43) Veröffentlichungstag der Anmeldung: **21.12.2005**
(21) Anmeldenummer: 05012761.2
(22) Anmeldetag: 14.06.2005
(51) Int. Cl.: A61B 17/14

(54) **Chirurgisches Sägeblatt**

(30) Priorität: 14.06.2004 DE 102004028691
(71) Anmelder: Gebr. Brasseler GmbH & Co. KG, D-32657 Lemgo (DE)
(72) Erfinder: Haverkamp, Siegfried, 32756 Detmold (DE); Danger, Karl-Heinz, 32758 Detmold (DE); Küllmer, Michael, 32657 Lemgo (DE); Otto, Berd, 32689 Kalletal (DE); Krumsiek, Michael, 32657 Lemgo (DE); Idel, Frank, 32657 Lemgo (DE); Meier, Friedrich Wilhelm, 32825 Blomberg (DE); Hagemann, Frank, 32657 Lemgo (DE); Brinkmann, Volker, 32657 Lemgo (DE)
(74) Vertreter: Weber, Joachim

(57) **Zusammenfassung**

Die Erfindung bezieht sich auf ein chirurgisches Sägeblatt mit einem plattenförmigen Körper, welcher mit einem Einspannbereich 1 und mit Sägezähnen 2 versehen ist, wobei beidseitig an dem plattenförmigen Körper 4 zumindest ein erhabener Bereich 3 ausgebildet ist, der mit einer Sägeschablone in Anlage bringbar ist, dadurch gekennzeichnet, dass der erhabene Bereich 3 durch Materialauftrag auf die Oberfläche des plattenförmigen Körpers 4 gebildet ist.

## Beschreibung

Die Erfindung bezieht sich auf ein chirurgisches Sägeblatt mit einem plattenförmigen Körper, welcher mit einem Einspannbereich und mit Sägezähnen versehen ist, nach dem Oberbegriff des Anspruches 1.

Chirurgische Sägeblätter werden verwendet, um während Operationen Knochen zu trennen. Typische Anwendungszwecke sind das Einsetzen von künstlichen Gelenken, beispielsweise Kniegelenken. Die Sägeblätter werden mittels Schablonen geführt, um einen möglichst passgenauen und präzisen Schnittverlauf zu realisieren.

Aus dem Stand der Technik ist es bekannt, Sägeblätter aus Stahl herzustellen, welche in passgenauen Schlitzen von Sägeschablonen geführt werden. Dabei versteht sich, dass der Schlitz der Schablone möglichst mit einem geringen Spiel versehen sein muss, um eine präzise, passgenaue Trennung des Knochens mittels des Sägeblattes sicherzustellen. Durch diese relativ enge Passung ergeben sich jedoch ungünstige Reibungsverhältnisse, welche zu einem nicht unbeachtlichen Abtrag von Material sowohl von dem Sägeblatt als auch von der Schablone führen kann. Diese= Abrieb ist aus medizinischen Gründen in hohem Maße unerwünscht, da die Metallpartikel sich in der Wunde festsetzen und zu allergischen Problemen, zu Problemen bei der Heilung und zu weiteren sonstigen medizinischen Komplikationen führen können.

Erschwerend kommt hinzu, dass unter den Operationsbedingungen keine ausreichende Spülung oder Schmierung möglich ist. Vielmehr setzen sich die Knochenspäne oder das Knochenmehl in dem Spalt zwischen dem Sägeblatt und der Schablone fest und erhöhen hierdurch zusätzlich den Verschleiß.

Durch den erhöhten Verschleiß an der Oberfläche der Sägeblätter werden auch dort angebrachte Markierungen, insbesondere Tiefenmarkierungen, unleserlich, sodass der Operateur vielfach nicht in der Lage ist, die bisherige Tiefe ausreichend zu kontrollieren.

Aus der DE 101 00 630 C1 ist ein Sägeblatt für eine chirurgische Oszillationssäge bekannt, bei welchem durch Materialabtrag Ausnehmungen vorgesehen sind, die streifenförmig oder geradlinig sein können. Hierdurch ergibt sich eine erhebliche Schwächung des Grundmaterials. Weiterhin führen die Ausnehmungen oder Nuten zu einem unterschiedlichen Festigkeitsverhalten des Sägeblatts selbst, welches in unerwünschten Schwingungen oder Verformungen resultiert.

Eine weitere Ausgestaltung eines Sägeblattes zeigt die EP 0 695 607 B1. Bei diesem Sägeblatt sind parallele Längsnuten ausgebildet, die ebenfalls zu einer Schwächung des Grundmaterials führen.

Der Erfindung liegt die Aufgabe zugrunde, ein chirurgisches Sägeblatt der eingangs genannten Art zu schaffen, welches bei einfachem Aufbau und kostengünstiger Herstellbarkeit die Nachteile des Standes der Technik vermeidet und dabei insbesondere den Materialabrieb reduziert.

Erfindungsgemäß wird die Aufgabe durch die Merkmalskombination des Hauptanspruchs gelöst, die Unteransprüche zeigen weitere vorteilhafte Ausgestaltungen der Erfindung.

Das erfindungsgemäße Sägeblatt weist somit an seinem plattenförmigen Körper zumindest einen erhabenen Bereich auf, der mit der Sägeschablone in Anlage bringbar ist.

Das chirurgische Sägeblatt zeichnet sich durch eine Reihe erheblicher Vorteile aus.

Durch den erhabenen Bereich, welcher gegen die Oberfläche des Schlitzes der Sägeschablone anliegt, ergibt sich eine verminderte Reibung, da die Auflagefläche minimiert ist. Durch die verminderte Reibung verringert sich entsprechend der Abrieb. Somit weist das erfindungsgemäße Sägeblatt (verglichen mit dem Stand der Technik) weitaus günstigere Eigenschaften hinsichtlich der Verschleißwerte und der unerwünschten Eintragung von Metallmaterial in die Operationsbereiche auf.

In einer besonders günstigen Ausgestaltung der Erfindung ist vorgesehen, dass beidseitig an dem plattenförmigen Körper zumindest ein erhabener Bereich ausgebildet ist. Dieser kann in bevorzugter Ausgestaltung in Form einer länglichen Rippe ausgebildet sein. Die Rippe kann sich dabei über die Längserstreckung des plattenförmigen Körpers des Sägeblattes ausdehnen, sodass das Sägeblatt bei Durchführung durch die Schablone im Laufe des Sägevorganges stets durch die erhabenen Bereiche geführt ist.

Der erhabene Bereich kann auch in Form einer Erhebung ausgebildet sein. Im Rahmen der Erfindung ergibt sich somit die Möglichkeit, entweder einen Materialauftrag aufzubringen, um den erhabenen Bereich zu erzeugen, oder im Zwischenbereich einen Materialabtrag vorzunehmen, sodass die erhabenen Bereiche als Rippen, Vorsprünge, Erhebungen oder Ähnliches vorstehen.

In einer besonders günstigen Variante der Erfindung ist es auch möglich, den erhabenen Bereich durch eine Verformung des plattenförmigen Körpers des Sägeblattes zu realisieren. Der plattenförmige Körper kann dabei geknickt oder geprägt werden, sodass sich Kanten ergeben, die den Kontakt zwischen dem Schlitz der Sägeschablone und dem Sägeblatt bilden. Dies wiederum führt zu einer besonders starken Reduzierung der tragenden Bereiche und damit der auftretenden Reibung.

Im Rahmen der Erfindung ist es möglich, die erhabenen Bereiche aus demselben Material zu fertigen, wie der plattenförmige Körper, beispielsweise durch die bereits beschriebenen Ausprägungen oder Verformungen oder durch einen Materialabtrag, der Rippen oder Ähnliches an den nicht abgetragenen Bereichen erzeugt. Es ist erfindungsgemäß jedoch auch möglich, die erhabenen Bereiche aus einem anderen Material zu fertigen, beispielsweise aus einem reibungsarmen Material oder einem verschleißfesten Material. Diese Materialien können galvanisch oder mittels eines Fügeverfahrens aufgebracht werden. Ebenso ist es möglich, die erhabenen Bereiche zusätzlich mit einer reibhemmenden Beschichtung zu versehen.

Bei bestimmten Formen von Sägeschablonen stößt das Sägeblatt zur Begrenzung seiner seitlichen Bewegung gegen Randbereiche eines Schlitzes oder einer Führung der Sägeschablone. Hierbei tritt beim Stand der Technik eine Gradbildung auf, welche unerwünscht ist, da die Gefahr besteht, dass sich Partikel dieses Grades lösen und in die Wunde gelangen. Weiterhin wird die Präzision des Sägeschnittes beeinträchtigt. Zusätzlich führt die Gradbildung zu einer zusätzlichen Reibung und kann zu einem Verklemmen des Sägeblattes in der Sägeschablone beitragen. In günstiger Ausgestaltung der Erfindung ist deshalb vorgesehen, dass seitliche Kanten des plattenförmigen Körpers mit einem verschleißfesten Material und/oder einem Hartmaterial versehen sind. Alternativ ist es auch möglich, die seitlichen Kanten in ihrer Dicke zu reduzieren. Bei einem Kontakt mit einer Sägeschablone kann sich somit ein geringfügiger Wulst aufbauen, ohne dass es zu einem Verklemmen kommt.

Um die Markierungen des Sägeblattes besser sichtbar zu machen und um deren Verschleiß zu minimieren, kann in Weiterbildung der Erfindung vorgesehen sein, dass benachbart zu den erhabenen Bereichen zumindest ein mit einer Markierung versehener Bereich vorgesehen ist. Dieser liegt somit tiefer und ist verschleißgeschützt, sodass die Markierung auch bei längerer Verwendung des Sägeblattes sich nicht abreibt und gut sichtbar ist.

Bei der Verwendung eines Materials für den erhabenen Bereich, welches gute Gleiteigenschaften und somit eine verminderte Reibung aufweist, ist es möglich, Kunststoffe einzusetzen. Es ist jedoch auch möglich, Keramik oder Hartmetall zu verwenden. Dabei versteht sich, dass diese Materialien, die die erhabenen Bereiche bilden, auch in Ausnehmungen oder Ausprägungen des Sägeblattes eingesetzt werden können, um zu einer sicheren Befestigung zu gelangen. So ist es beispielsweise möglich, Kunststoffnoppen oder Keramiknoppen in passende Ausnehmungen des plattenförmigen Körpers des Sägeblattes einzusetzen.

Im Folgenden wird die Erfindung anhand von Ausführungsbeispielen in Verbindung mit der Zeichnung beschrieben. Dabei zeigt:
- Fig. 1: eine Seitenansicht eines ersten Ausführungsbeispiels des erfindungsgemäßen Sägeblattes mit erhabenen Bereichen, welche durch Materialabtrag erzeugt wurden,
- Fig. 2: eine alternative Ausgestaltungsform, analog dem in Fig. 1 gezeigten Ausführungsbeispiel,
- Fig. 3: eine weitere Ausgestaltungsvariante mit erhabenen Bereichen aus einem anderen Material als der plattenförmige Körper,
- Fig. 4: eine weitere Ausgestaltungsvariante, ähnlich dem Ausführungsbeispiel der Fig. 3,
- Fig. 5: eine weitere Ausgestaltungsvariante, ähnlich den Ausführungsbeispielen der Fig. 3 und 4,
- Fig. 6: eine Ausgestaltungsvariante mit eingesetzten, die erhabenen Bereiche bildenden Noppen,
- Fig. 7: ein Ausgestaltungsbeispiel eines Sägeblatts mit einem balligen Querschnitt,
- Fig. 8: ein Ausgestaltungsbeispiel eines verformten Sägeblatts,
- Fig. 9: eine weitere Ausgestaltungsvariante eines mit einem verformten plattenförmigen Körper versehenen Sägeblattes,
- Fig. 10: eine Seitenansicht eines weiteren Ausführungsbeispiels des erfindungsgemäßen Sägeblatts, und
- Fig. 11: ein weiteres Ausführungsbeispiel, ähnlich dem der Fig. 10.

Bei den nachfolgenden Ausführungsbeispielen sind gleiche Teile jeweils mit gleicher Bezugsziffer versehen.

Die gezeigten Ausführungsbeispiele umfassen jeweils einen im Wesentlichen plattenförmigen Körper 4, welcher länglich ausgebildet ist und an seinem einen Endbereich mit einem Einspannbereich 1 versehen ist. Die Ausgestaltung des Einspannbereichs 1 entspricht dem Stand der Technik und ist angepasst an die jeweilige Antriebseinrichtung, mit welcher das chirurgische Sägeblatt zu verwenden ist.

Gegenüberliegend zu dem Einspannbereich 1 sind eine Vielzahl von Sägezähnen 2 ausgebildet, die auf einem Kreisbogen angeordnet sein können, dessen Zentrum in der nicht dargestellten Drehachse des Einspannbereichs 1 liegt.

Die Dimensionierung der erfindungsgemäßen Sägeblätter entspricht dem Stand der Technik und ist an die nicht im Einzelnen dargestellten und erläuterten Sägeschablonen angepasst.

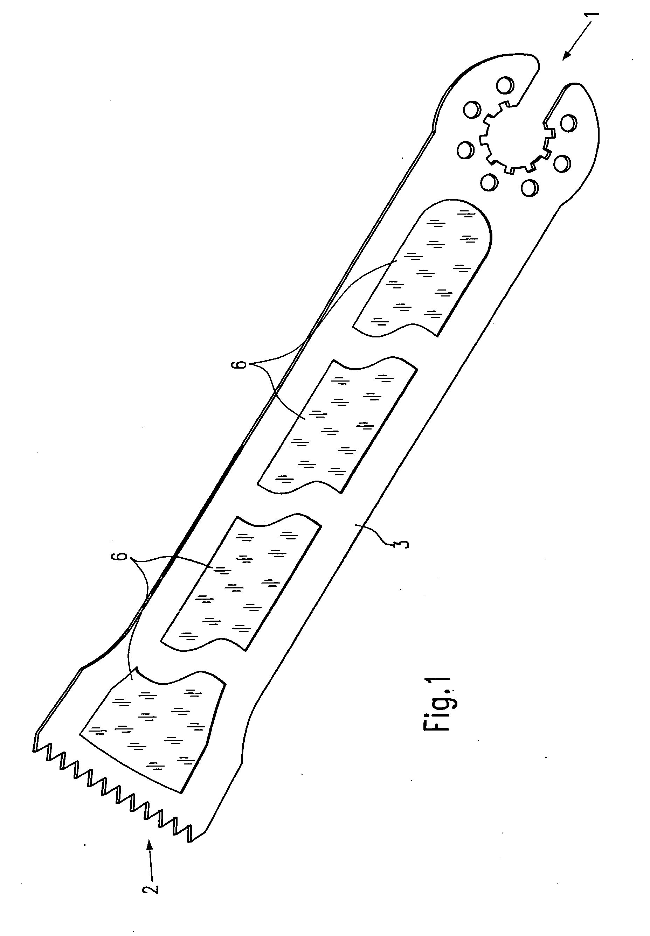

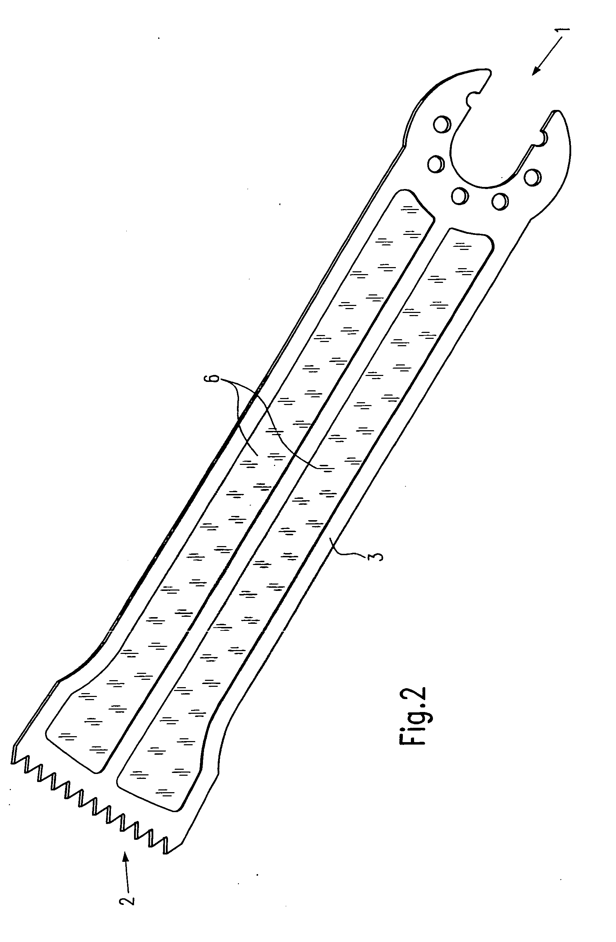

Bei den in den Fig. 1 und 2 gezeigten Ausführungsbeispielen sind an dem plattenförmigen Körper 4 Vertiefungen 6 vorgesehen, die beispielsweise durch einen Ätzvorgang erzeugt werden können. Somit verbleiben leistenförmige oder rippenförmige erhabene Bereiche 3, die zur Anlage an einen Schlitz einer Sägeschablone kommen. Wie aus den Fig. 1 und 2 ersichtlich ist, führt dies zu einer erheblichen Verminderung der Kontaktfläche zu einer Sägeschablone und damit zu einem verminderten Abrieb. Es versteht sich, dass ein verminderter Abrieb sowohl an dem Sägeblatt als auch an der Schablone auftritt. Insofern addieren sich die günstigen Effekte.

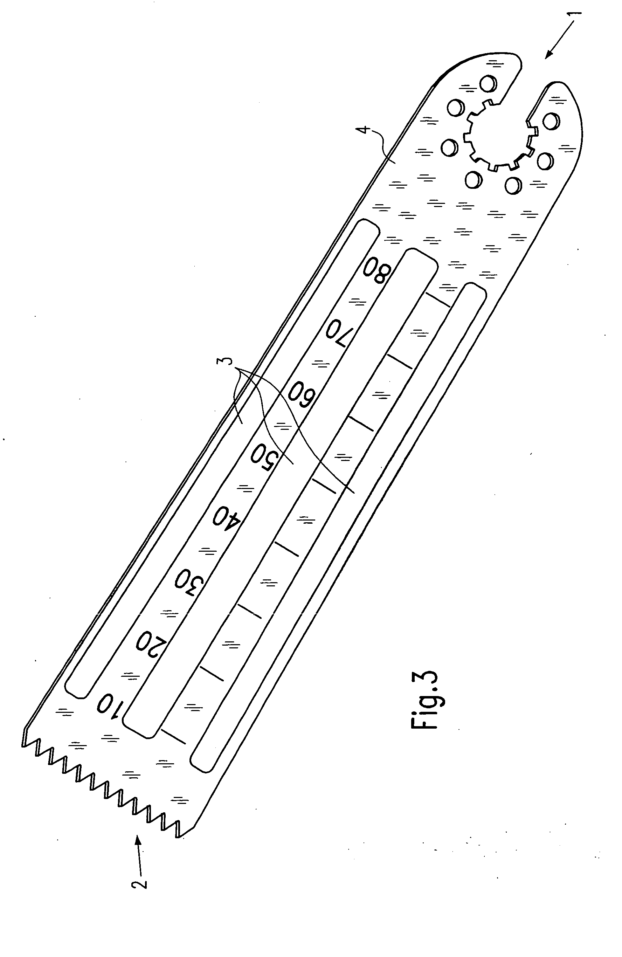

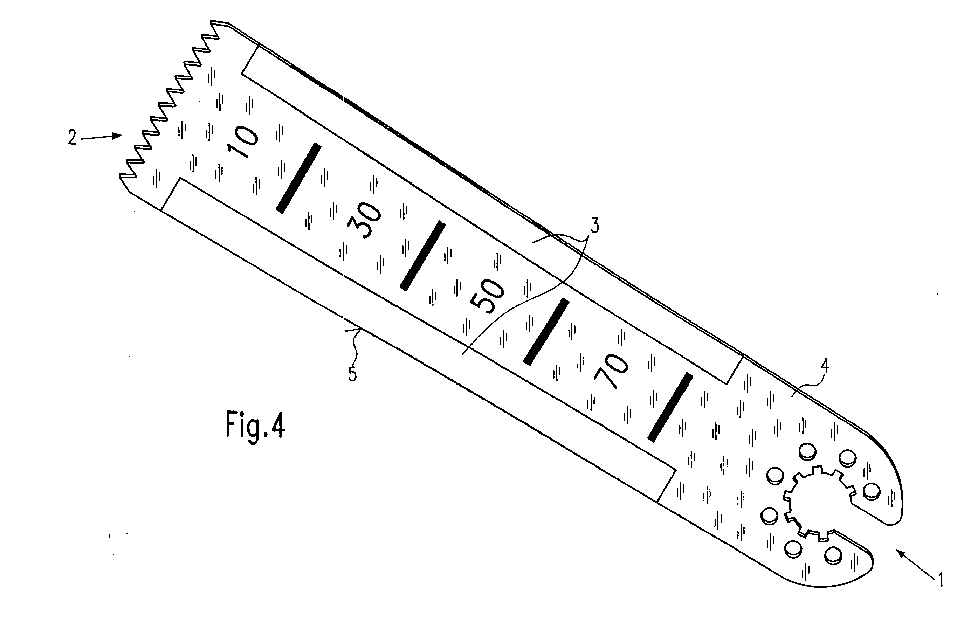

Bei den Ausführungsbeispielen der Fig. 3 bis 5 sind die erhabenen Bereiche 3 dadurch realisiert, dass Material aufgebracht ist. Dieses kann in Form von Gleitschienen auf der Oberfläche des plattenförmigen Körpers 4 befestigt sein. Die Gleitschienen können, wie bei den Ausführungsbeispielen der Fig. 3 und 4, rippenförmig bzw. in Form von schmalen Streifen vorgesehen sein. In den zwischen den erhabenen Bereichen 3 verbleibenden Oberflächenbereichen des plattenförmigen Körpers 4 können Beschriftungen oder Markierungen aufgebracht werden, die vor Verschleiß und Abrieb geschützt sind und somit auch nach längerem Einsatz des Sägeblattes gut lesbar sind.

Es versteht sich, dass bei sämtlichen der gezeigten Ausführungsformen der Dickenunterschied zwischen den erhabenen Bereichen 3 und der Oberfläche des plattenförmigen Körpers 4 dazu führt, dass sich ein zusätzlicher Raum ergibt, durch welchen Knochenspäne oder Knochenmehl abtransportiert werden können. Hierdurch wird verhindert, dass sich das Sägeblatt in der Sägeschablone verklemmt. Dies ist auch im Hinblick darauf wichtig, dass bei derartigen Operationen die Sägespäne oder das Knochenmehl durch Blut oder andere Flüssigkeiten verkleben oder verklumpen. Insgesamt ergibt sich somit ein weitaus verbessertes Sägeverhalten als bei den aus dem Stand der Technik bekannten Sägeblättern.

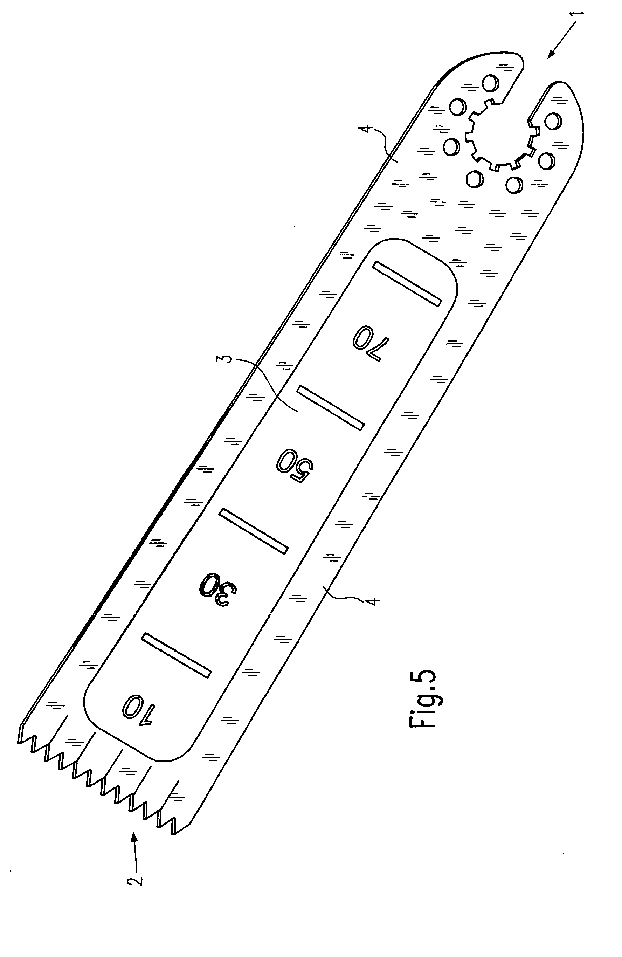

Das in Fig. 5 gezeigte Ausführungsbeispiel sieht einen breiteren, mittigen Streifen eines aufgebrachten Materials vor, welcher als Gleitfläche dient. Durch Ausprägungen kann die gezeigte Markierung realisiert werden. Die Randbereiche des Sägeblattes sind bei dem in Fig. 5 gezeigten Ausführungsbeispiel somit nicht-tragend.

Um eine Beschädigung der Kanten des Sägeblattes zu vermeiden, ist bei dem in Fig. 4 gezeigten Ausführungsbeispiel vorgesehen, dass die seitliche Kante 5 durch die erhabenen Bereiche überdeckt wird. Sofern diese aus einem verschleißfesten Material bestehen, tritt keine oder nur eine deutlich reduzierte Wulstbildung beim Anschlag der seitlichen Kante 5 gegen die Sägeschablone auf.

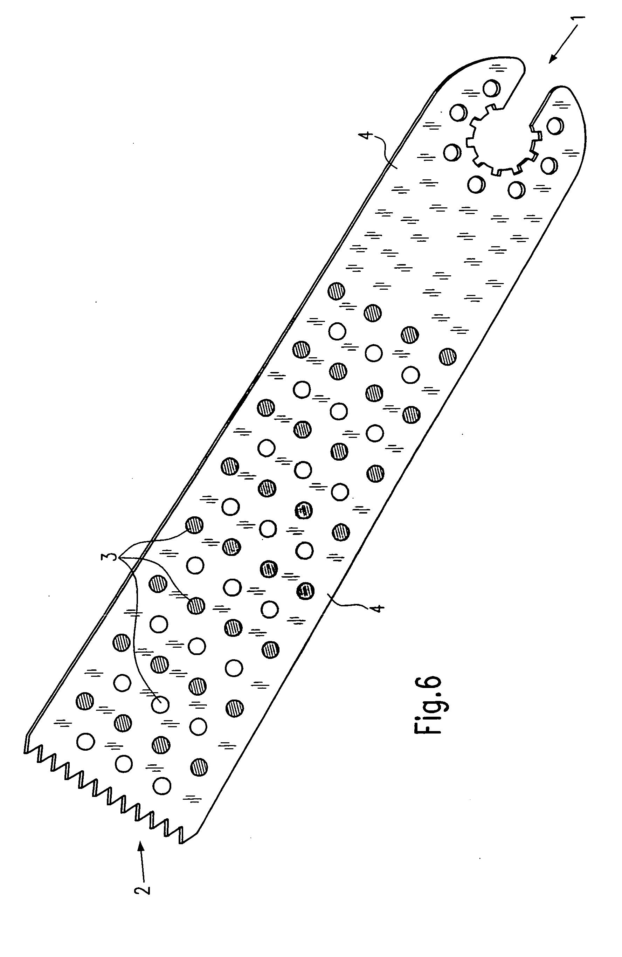

Bei dem Ausführungsbeispiel der Fig. 6 weist der plattenförmige Körper 4 eine Vielzahl von punktuellen erhabenen Bereichen 3 auf. Diese können beispielsweise durch eingesetzte Noppen oder Ähnliches oder durch aufgesetzte, aufgeklebte oder in anderer Weise befestigte erhabene Bereiche 3 gebildet werden. Auch hierdurch ergibt sich eine deutliche Reduzierung der tragenden Oberfläche.

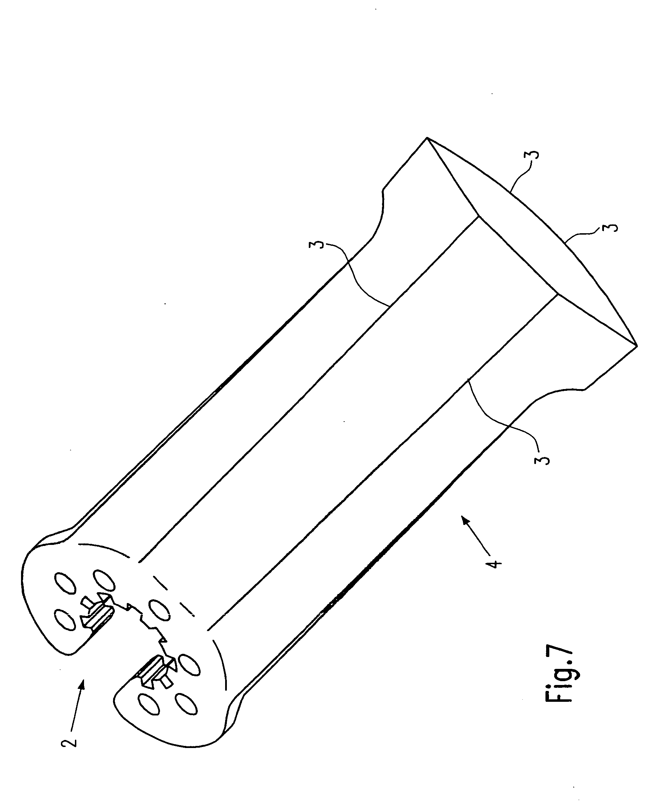

Das Ausführungsbeispiel der Fig. 7 zeigt eine ballige oder konvexe Grundgestalt und Querschnittsform des plattenförmigen Körpers 4. Diese weist somit eine plane mittlere Fläche auf, an welcher zwei Kanten vorgesehen sind, welche die erhabenen Bereiche bilden. Die Ränder sind mit einem deutlich verminderten Querschnitt versehen, sodass dort keine Kontaktierung mit der Sägeschablone stattfindet. Es versteht sich, dass zwischen den parallel verlaufenden Kanten der erhabenen Bereiche 3 auch eine konkave Oberfläche möglich ist, sodass lediglich die vier gezeigten kantenförmigen erhabenen Bereiche 3 im Kontakt mit der Sägeschablone kommen. Bei seitlichem Kontakt zur Schablone verursachen die entstehenden Aufwerfungen keine Reibung in der Schablone, da der Grundkörper 4 dicker ist.

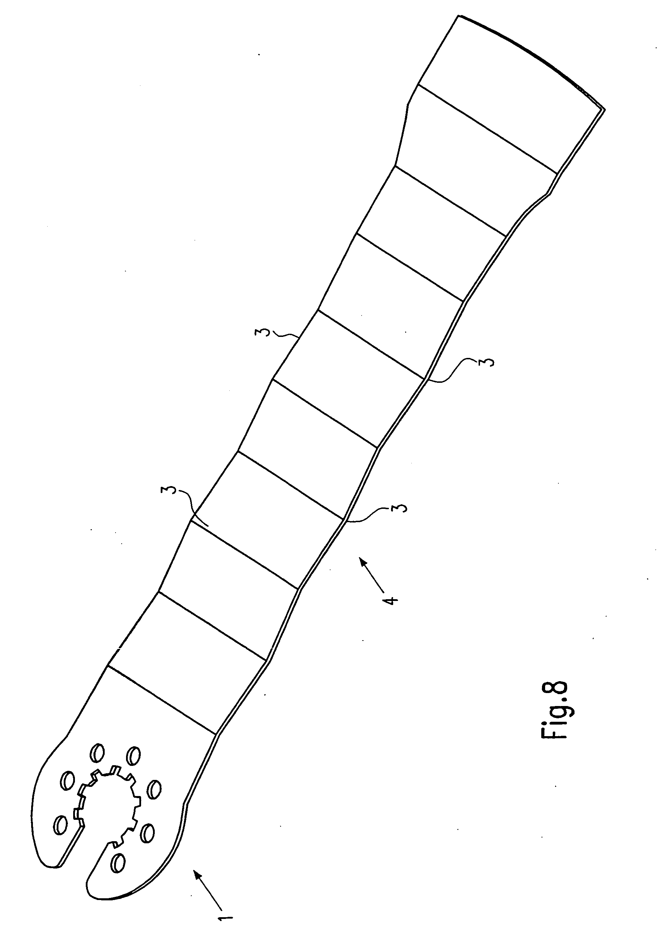

Bei dem in Fig. 8 gezeigten Ausführungsbeispiel ist der plattenförmige Körper 4 quer zu seiner Längsrichtung alternierend geknickt. Hierdurch ergeben sich Kanten, welche die erhabenen Bereiche 3 bilden. Abhängig von der Größe der Sägeschablone tragen gleichzeitig jeweils mehrerer dieser erhabenen Kanten 3, sodass sich eine stabile Führung des Sägeblattes ergibt. Es liegt somit lediglich eine Linienberührung zu der Sägeschablone vor, sodass sich die Reibungsfläche deutlich reduziert.

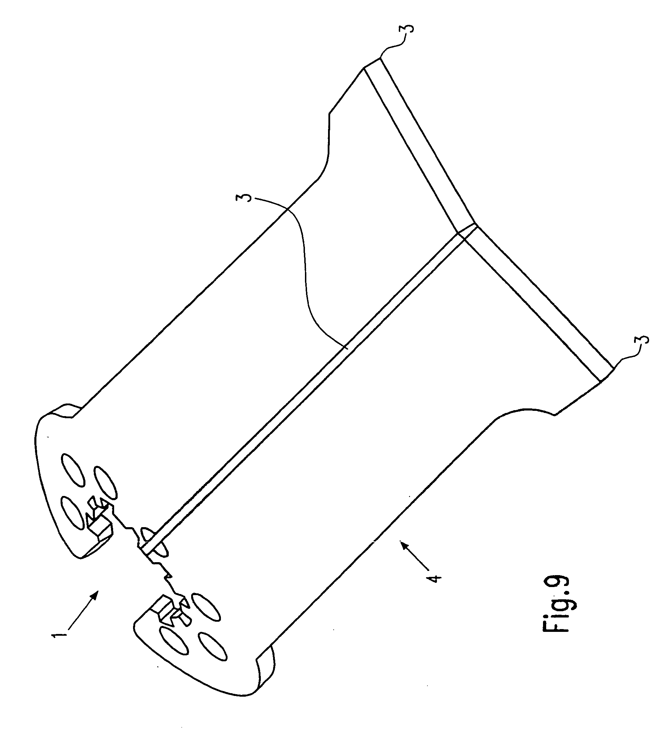

Die Fig. 9 zeigt ein weiteres Ausführungsbeispiel, bei welchem der erhabene Bereich durch eine Längsfaltung des plattenförmigen Körpers 4 ergibt. Somit ergibt sich eine Linienführung des mittigen erhabenen Bereichs 3 sowie der beiden seitlichen Kanten.

Bei den Ausführungsbeispielen der Fig. 7 bis 9 wurde auf die Darstellung der Sägezähne 2 verzichtet, insbesondere bei den Ausführungsbeispielen der Fig. 7 und 9 wurde nur ein Teilbereich der Gesamtlänge des Sägeblattes gezeigt.

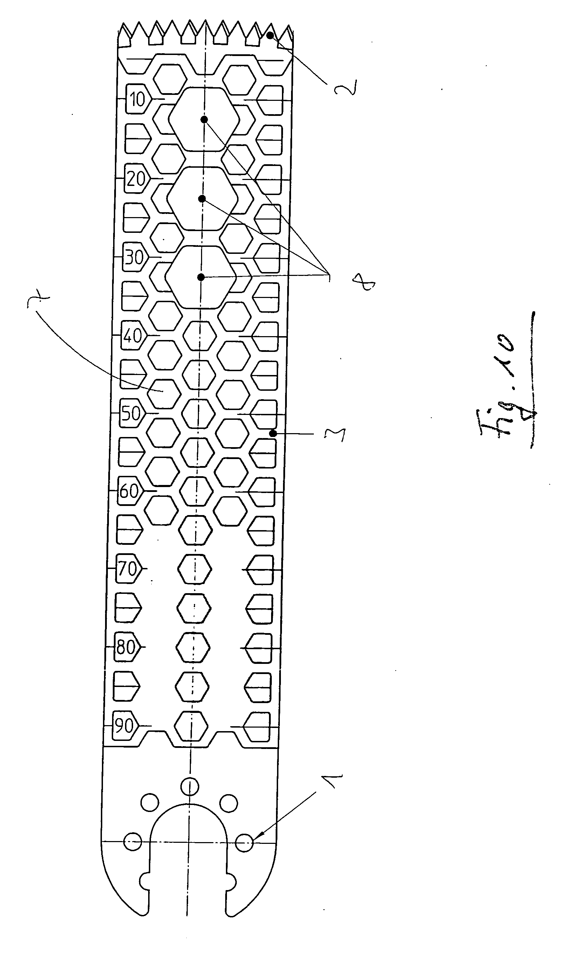

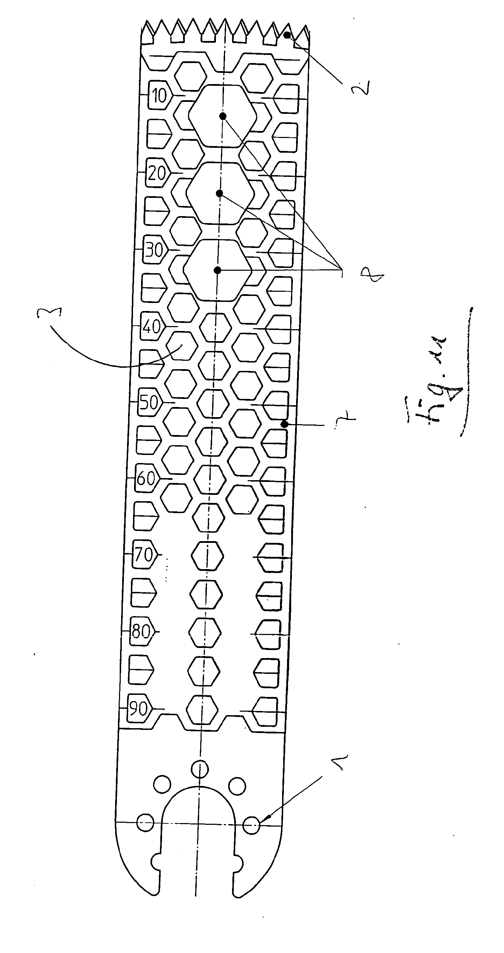

Die Fig. 10 und 11 zeigen Ausführungsbeispiele, bei welchen sich eine wabenartige Struktur von erhabenen Bereichen 3 und nicht-erhabenen Bereichen 7 ergibt. Unter wabenförmiger Struktur wird nicht eine Beschränkung auf sechseckige Waben verstanden, vielmehr handelt es sich dabei um Anordnungen geometrischer, zueinander gleicher Flächen, welche jeweils in geradliniger Orientierung angeordnet sind und bei denen die einzelnen Reihen zueinander versetzt sind.

Die Fig. 10 zeigt eine Ausgestaltung, bei welcher die erhabenen Bereiche in Form einer regelmäßigen, gitterartigen Stegstruktur ausgebildet sind und jeweils sechseckige, nicht-erhabene Bereiche 7 einschließen. Zusätzlich sind angrenzend an den vorderen, mit den Sägezähnen 2 versehenen Bereich Ausnehmungen 8 ausgebildet, welche Durchbrüche darstellen und insbesondere zur Spanabfuhr dienen.

Bei dem Ausführungsbeispiel der Fig. 11 ist eine Inversion der Ausgestaltung der Fig. 10 vorgenommen worden. Diesbezüglich sind somit die erhabenen Bereiche 3 in Form sechseckiger geometrischer Objekte ausgebildet. In den Randbereichen sind die erhabenen Bereiche lediglich fünfeckig, um eine gleichmäßige Anlagestruktur schaffen zu können. Auch bei diesem Ausführungsbeispiel sind die Ausnehmungen 8 vorgesehen.

Es versteht sich, dass die bei den Ausführungsbeispielen gezeigten Ausgestaltungen jeweils beidseitig an dem plattenförmigen Körper vorgesehen sind, sodass sich zu beiden Seiten des Sägeblattes eine entsprechende reibungsvermindernde Führung der Sägeschablone ergibt.

### Bezugszeichenliste

- 1: Einspannbereich
- 2: Sägezahn
- 3: Erhabener Bereich
- 4: Plattenförmiger Körper
- 5: Seitliche Kante
- 6: Vertiefung
- 7: Nicht-erhabener Bereich
- 8: Ausnehmung

## Patentansprüche

1. Chirurgisches Sägeblatt mit einem plattenförmigen Körper, welcher mit einem Einspannbereich (1) und mit Sägezähnen (2) versehen ist, wobei beidseitig an dem plattenförmigen Körper (4) zumindest ein erhabener Bereich (3) ausgebildet ist, der mit einer Sägeschablone in Anlage bringbar ist, **dadurch gekennzeichnet, dass** der erhabene Bereich (3) durch Materialauftrag auf die Oberfläche des plattenförmigen Körpers (4) gebildet ist.

2. Sägeblatt nach Anspruch 1, **dadurch gekennzeichnet, dass** der erhabene Bereich (3) in Form einer länglichen Rippe ausgestaltet ist.

3. Sägeblatt nach Anspruch 1, **dadurch gekennzeichnet, dass** der erhabene Bereich (3) in Form einer Erhebung ausgestaltet ist.

4. Sägeblatt nach einem der Ansprüche 1 bis 3, **dadurch gekennzeichnet, dass** der erhabene Bereich (3) in Form einer Gitterstruktur ausgebildet ist, welche wabenförmig nicht-erhabene Bereiche (7) umschließt.

5. Sägeblatt nach Anspruch 4, **dadurch gekennzeichnet, dass** die nicht-erhabenen Bereiche (7) in zueinander versetzten parallelen Reihen angeordnet sind.

6. Sägeblatt nach Anspruch 4 oder 5, **dadurch gekennzeichnet, dass** die nicht-erhabenen Bereiche (7) im Wesentlichen sechseckig ausgebildet sind.

7. Sägeblatt nach einem der Ansprüche 1 bis 3, **dadurch gekennzeichnet, dass** die erhabenen Bereiche (3) in gleichmäßiger Anordnung in zueinander versetzten Reihen angeordnet und zueinander beabstandet sind.

8. Sägeblatt nach Anspruch 7, **dadurch gekennzeichnet, dass** die erhabenen Bereiche (3) im Wesentlichen sechseckig ausgebildet sind.

9. Sägeblatt nach einem der Ansprüche 1 bis 8, **dadurch gekennzeichnet, dass** der erhabene Bereich (3) durch Verformung des plattenförmigen Körpers (4) gebildet ist.

10. Sägeblatt nach einem der Ansprüche 1 bis 9, **dadurch gekennzeichnet, dass** der erhabene Bereich (3) aus demselben Material wie der plattenförmige Körper (4) gefertigt ist.

11. Sägeblatt nach einem der Ansprüche 1 bis 10, **dadurch gekennzeichnet, dass** der erhabene Bereich (3) aus einem reibungsarmen Material gefertigt ist.

12. Sägeblatt nach einem der Ansprüche 1 bis 11, **dadurch gekennzeichnet, dass** der erhabene Bereich (3) aus einem verschleißfesten Material gefertigt ist.

13. Sägeblatt nach einem der Ansprüche 1 bis 12, **dadurch gekennzeichnet, dass** seitliche Kanten (5) des plattenförmigen Körpers (4) mit einem verschleißfesten und/oder einem Hartmaterial versehen sind.

14. Sägeblatt nach einem der Ansprüche 1 bis 13, **dadurch gekennzeichnet, dass** benachbart zu dem erhabenen Bereich (3) zumindest ein mit einer Markierung versehener Bereich vorgesehen ist.

15. Sägeblatt nach einem der Ansprüche 1 bis 14, **dadurch gekennzeichnet, dass** seitliche Kanten (5) des plattenförmigen Körpers (4) eine reduzierte Dicke aufweisen.

16. Sägeblatt nach einem der Ansprüche 1 bis 15, **dadurch gekennzeichnet, dass** benachbart zu dem erhabenen Bereich (3) zumindest ein mit einer Markierung versehener Bereich vorgesehen ist.
